# EUROPEAN PATENT APPLICATION

(11) **EP 4 664 400 A1**
(43) Date of publication of application: **17.12.2025**
(21) Application number: 24182264.2
(22) Date of filing: 14.06.2024
(51) Int. Cl.: G06T 7/246

(54) **SUBJECT TRACKING**

(71) Applicant: Koninklijke Philips N.V., 5656 AG Eindhoven (NL)
(72) Inventor: TERMEER, JMaurice Alain, Eindhoven (NL); HOMAN, Robert Johannes Frederik, Eindhoven (NL)
(74) Representative: Philips Intellectual Property & Standards

(57) **Abstract**

The present invention relates to tracking a subject, for example during medical examinations, interventions or other medical procedures. In order to provide improved subject tracking, a device (10) for subject tracking is provided that comprises a data input (12), a data processor (14) and an output interface (16). The data input is configured: to provide image data (18) of a subject comprising representation of at least four identifiable markers attached to the subject; and to provide a 3D reference model (20) of the markers which is based on an initial spatial arrangement of the markers attached to the subject. The data processor is configured: to identify the markers and their positions; to extract a marker arrangement based on the identified markers and their positions; to search for a spatial positioning of the 3D reference model matching with a maximum subset of markers of the extracted marker arrangement; to identify markers of the extracted marker arrangement that are not part of the matching subset as temporarily ignored; and to determine the spatial positioning of the 3D reference model that is matching with the maximum subset of the markers. The output interface is configured to provide the spatial orientation of the 3D reference model for a calculation of a spatial position of the subject.

## Description

### FIELD OF THE INVENTION

The present invention relates to tracking a subject, for example during medical examinations, interventions or other medical procedures. The present invention relates in particular to a device for subject tracking, to a subject tracking kit, to a subject tracking system and to a method for subject tracking.

### BACKGROUND OF THE INVENTION

For medical interventions, subject tracking is provided, for example for combining different image data such as pre-interventional image data with live image data. An example is to be able to visualize the location of instruments. Tracking systems, e.g. optical tracking systems, can be used as a navigation system during surgery. For example, a navigation system provides real-time information on the location of a surgical instrument relative to the patient. This can be especially useful during minimal invasive image guided interventions, which are more and more replacing open surgery procedures. One task of the navigation system is to track the patient in space and time and compensate for any patient moment when visualizing the location of instruments. A common solution is to attach one or more markers that can easily be tracked by the tracking system to the patient. The marker (or set of markers) must provide full six degree-of-freedom information on the position and orientation of the patient. An example of such a marker is a metal frame that houses several reflective spheres that is screwed into the bone of the patient. This type of marker is rigid and offers robust tracking, but attaching to the patient may be cumbersome. Another type of marker consists of stickers that are placed on the skin of the patient around the area of interest for the surgery. The positions of all stickers can be combined to provide six degree-of-freedom tracking on the patient. However, it has been shown that the markers attached to the skin are prone to errors. For example, individual markers can accidentally move on the skin or even detach from the subject.

### SUMMARY OF THE INVENTION

There may thus be a need to provide improved subject tracking.

The object of the present invention is solved by the subject-matter of the independent claims; further embodiments are incorporated in the dependent claims. It should be noted that the following described aspects of the invention apply also for the device for subject tracking, for the subject tracking kit, for the subject tracking system and for the method for subject tracking.

According to the present invention, a device for subject tracking is provided. The device comprises a data input, a data processor and an output interface. The data input is configured to provide image data of a subject comprising representation of at least four identifiable markers attached to the subject. The data input is also configured to provide a 3D reference model of the markers which is based on an initial spatial arrangement of the markers attached to the subject. The data processor is configured to identify the markers and their positions. The data processor is also configured to extract a marker arrangement based on the identified markers. The data processor is further configured to search for a spatial positioning of the 3D reference model matching with a maximum subset of markers of the extracted marker arrangement. The data processor is furthermore configured to identify markers of the extracted marker arrangement that are not part of the matching subset as temporarily ignored. The data processor is configured to determine the searched spatial positioning of the 3D reference model that is matching with the maximum subset of the markers. The output interface is configured to provide the spatial orientation of the 3D reference model for a calculation of a spatial position of the subject.

As an effect, the proposed tracking of the position and orientation of a subject using multiple markers is robust against one or more of the patient markers gradually moving relative to the other markers during the procedure. The tracking is also robust against one or more patient markers being occluded, e.g. temporarily not visible. The tracking is furthermore robust against one or more objects that are not patient markers that are observed as such by the navigation system, so-called false positives. Robust against means that the tracking is able to provide reliable information about the position of the subject if these instances occur.

As a particular advantage, even the gradual movement of markers is addressed.

According to an example, the data processor is further configured to compute the spatial position of the subject based on the identified spatial orientation of the 3D reference model.

According to an example, the data input is configured to provide image data comprising spatial information for the markers. The data processor is configured to extract a 3D marker arrangement based on the identified markers and their positions and the spatial information for the markers; and to search for a spatial positioning of the 3D reference model matching with a maximum subset of markers of the extracted 3D marker arrangement; and to determine a spatial orientation of the 3D reference model for the searched spatial positioning that is matching with the maximum subset of the markers.

This provides the advantage that the search for the best match can be arranged within an imaginary, i.e. virtual, 3D space.

In an option, the data input is configured to provide image data comprising 2D images of the markers from at least two directions. The data processor is configured to generate the 3D marker arrangement based on the 2D images of the markers from the at least two directions.

As an advantage, facilitated imaging can be applied. Even though the 2D images themselves miss the depth information, by providing the imaging direction relation with one or more further image, the missing part of the depth is at least essentially compensated for. However, the three-dimensional information is still present during the best-matching of the detected markers and the reference model. The search for the best match is arranged within the imaginary, i.e. virtual, 3D space.

In another option, the data input is configured to provide image data comprising 3D image data of the markers. The data processor is configured to generate the 3D marker arrangement based on the 3D image data of the markers.

This reduces the need to compute, i.e. reconstruct the 3D information since the depth information is already immanent within the 3D image data. The search for the best match is arranged within the imaginary, i.e. virtual, 3D space.

According to an example, the data input is configured to provide a 2D image. The data processor is configured to extract a 2D marker arrangement based on the identified markers and their positions; and to search for a 2D projection of the 3D reference model matching with a maximum subset of markers of the extracted marker arrangement; and to identify the spatial orientation of the 3D reference model based on the searched 2D projection.

As an advantage, the image data can be generated with facilitated imaging means, such as a single camera, or in cases where a second camera's view is occluded. However, the depth information is provided by the known projecting information of the matching projection of the 3D model. The search for the best match is arranged within the 2D image plane, i.e. not directly in a 3D space, but the unique projection of a marker arrangement allows to reconstruct the viewing direction for that projection and thus supplements the missing spatial information.

According to an example, the data input is configured to provide image data relating to at least one further point in time. The data processor is further configured to repeat the steps of marker identification, marker extraction, search of a projection matching with a maximum subset of markers and determination of the searched projection for a calculation to track a movement of the subject, based on the image data relating to at least one further point in time.

In an example, the data input is configured to provide 2D or 3D image data relating to at least one further point in time.

According to an example, the data processor is further configured to compute a current spatial position of the subject based on the identified spatial orientation of the 3D reference model.

According to an example, the provided image data of the subject relates to a first point in time. The data processor is further configured to temporarily identify the temporarily ignored markers as displaced markers. The data input is configured to provide image data from at least one further point in time. Further, the data processor is configured to detect if the markers previously identified as temporarily ignored markers are having a stable position. The data processor is furthermore configured to identify stable markers of the temporarily ignored markers as shifted markers. The data processor is also configured to update the 3D reference model based on the shifted markers and to apply the updated 3D reference model for further projection searching steps.

In an example, the provided image data of the subject relating to a first point in time is 2D or 3D image data.

According to an example, for the provision of the 3D reference model, the data input is configured to provide initial image data of the markers attached to the subject. Further, the data processor is further configured to determine the 3D model as an initial 3D model based on the initial image data, the 3D model representing an initial spatial arrangement of the markers.

According to the present invention, also a subject tracking kit is provided. The kit comprises a subject tracking device according to one of the examples above. The kit also comprises a set of at least four markers. The markers are configured to be attached to a subject's outer surface. The markers are detectable by a marker detection device outputting image data to the subject tracking device, the image data comprising representations of the markers attached to the subject.

According to an example, in a first option, the markers are markers detectable by a camera. In a second option, in addition or alternatively, the markers are markers detectable by at least one of the group comprising a depth camera using time-of-flight sensors, a LIDAR sensor and a structural light sensor.

According to an example, the markers are electromagnetic markers detectable by an electromagnetic tracking system.

According to an example, in a first option, the markers are to be provided on the subject in a non-symmetric spatial arrangement. In a second option, alternatively or additionally, the markers are to be provided on the subject with different distances between the markers.

According to the present invention, also a subject tracking system is provided. The system comprises a marker detection arrangement comprising at least one marker detection device. The system also comprises a subject tracking device or a subject tracking kit according to one of the examples above. The at least one marker detection device is configured to output image data to the subject tracking device, the image data comprising representations of the markers attached to the subject.

In an example, the at least one marker detection device is configured to output 2D or 3D image data to the subject tracking device, the 2D or 3D image data comprising representations of the markers attached to the subject.

According to the present invention, also a method for subject tracking is provided. The method comprising the following steps:
- Providing image data of a subject comprising representations of at least four identifiable markers attached to the subject;
- Identifying the markers and their positions;
- Extracting a marker arrangement based on the identified markers and their positions;
- Providing a 3D reference model of the markers which is based on an initial spatial arrangement of the markers attached to the subject;
- Searching for a projection of the 3D reference model matching with a maximum subset of markers of the extracted marker arrangement;
- Identifying markers of the extracted marker arrangement that are not part of the matching subset as temporarily ignored;
- Determining the searched projection of the 3D reference model that is matching with the maximum subset of the markers and identifying a spatial orientation of the 3D reference model based on the projection direction; and
- Providing the spatial orientation of the 3D reference model for a calculation of a spatial position of the subject.

In an example, the image data is 3D image data. The markers are identified with their 3D positions.

In another example, the image data is 2D image data. The markers are identified with their 2D positions. As an example, for the conversion of detected 2D marker position to 3D positions, multiple optical cameras are provided to detect the 2D positions, triangulate them to 3D position, and use the result to establish the relation with a previously stored reference model.

In an example of the method, the provided image data of the subject comprises spatial information for the markers. It is further provided the step of extracting a 3D marker arrangement based on the identified markers and their positions and the spatial information for the markers. It is still further provided the step of searching for a spatial positioning of the 3D reference model matching with a maximum subset of markers of the extracted 3D marker arrangement. It is furthermore provided the step of determining a spatial orientation of the 3D reference model for the searched spatial positioning that is matching with the maximum subset of the markers.

In a first option of the method, the provided image data of the subject comprises 2D images of the markers from at least two directions. It is further provided the step of generating the 3D marker arrangement based on the 2D images of the markers from the at least two directions.

In a second option of the method, the provided image data of the subject comprises 3D image data of the markers. It is further provided the step of generating the 3D marker arrangement based on the 3D image data of the markers.

In another example of the method, the provided image data of the subject comprises only a 2D image. It is further provided the step of:
extracting a 2D marker arrangement based on the identified markers and their positions. It is still further provided the step of searching for a 2D projection of the 3D reference model matching with a maximum subset of markers of the extracted marker arrangement. It is furthermore provided the step of identifying the spatial orientation of the 3D reference model based on the searched 2D projection.

The present invention proposes tracking of a subject using multiple skin markers that is robust against such slight movement of markers. It is also robust against partial occlusions of the set of markers and false positives.

According to an aspect, patient tracking using skin markers is provided. The invention proposes tracking the position and orientation of a patient using a collection of skin markers. The method is robust against movement of individual markers by tracking the largest matching subset of markers. In an option, updating the positions of moving markers once they are stable again is provided.

As an advantage, the navigation system can track a subject even if only a subset of the markers is visible. The same approach, i.e. the same mechanism, allows the system to still track the patient if a subset of the patient markers is deformed.

The navigation system searches for the largest subset of observed markers that matches with the stored reference model with an acceptable quality. In an example, the acceptable quality relates to any deviation below a predetermined threshold.

According to an aspect, a mode of tracking is provided in which markers are identified to be temporarily ignored.

According to an aspect, markers are provided on the subject which markers can be identified in image data, such as 2D or 3D image data, e.g. the markers can visually be detected in camera image data. A 3D model is created representing the markers. In an option, the 3D model is based on an initial registration of subject 3D data and initial images taken from the markers. The 3D model is used to provide a plurality projections of markers of the model to search for a match with a current image in which the markers are provided, e.g. in a 3D image space or projected in the 2D image plane. The matching is done with the approach to identify the best matching for the highest number of markers. This takes into account the possibility that one or more of the markers have moved in comparison to the initial arrangement. An example for moving markers are skin portions that change e.g. due to an incision. Markers can also accidently move on the skin portion. Another example for moving markers are sagging subject portions, such as when resting on a subject support over a longer period of time.

As an option, when markers are moved during the procedure, disappear from view, etc., the navigation system can track a patient if only a subset of markers is visible. In particular, the navigation system searches for the largest subset of observed markers that matches with the stored reference model with an acceptable quality, i.e. any deviation is below a predetermined threshold).

The present solution provides an adapted tracking that compensates for shifted or otherwise de-placed markers.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for subject tracking.
Fig. 2 shows an example of an arrangement of attached markers as tracked by a tracking device.
Fig. 3a, 3b and 3c show the arrangement of the attached markers of Fig. 2 in three different states illustrating an example of a scheme of subject tracking.
Fig. 4 shows an example of a subject tracking kit
Fig. 5 shows an example of a subject tracking system.
Fig. 6 shows basic steps of an example of a method for subject tracking.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

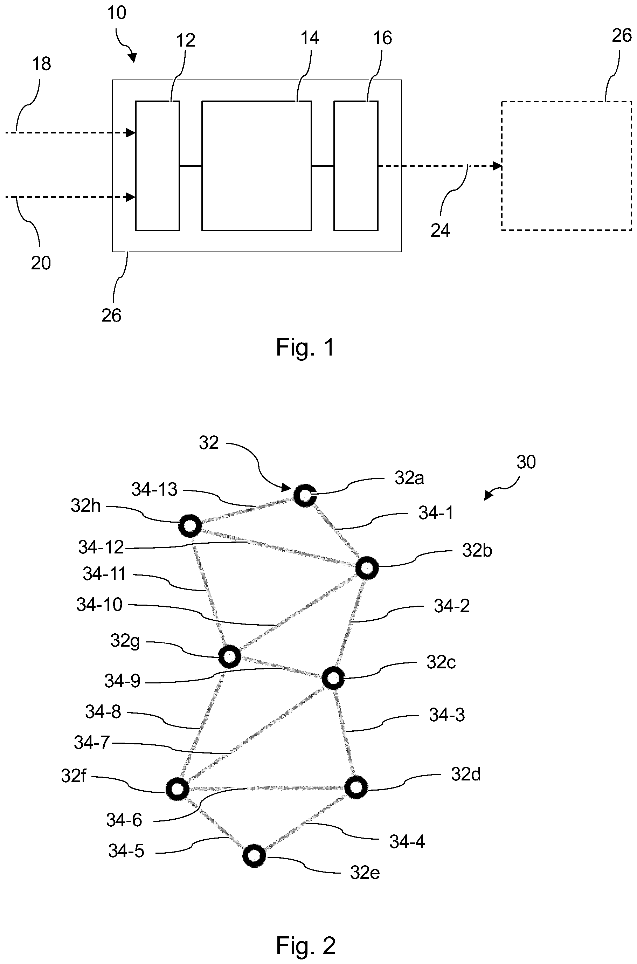

Fig. 1 schematically shows an example of a device 10 for subject tracking. The device 10 comprises a data input 12, a data processor 14 and an output interface 16. The data input 12 is configured to provide image data 18 (indicated with a first broken-line arrow) of a subject comprising representation of at least four identifiable markers attached to the subject. The data input 12 is also configured to provide a 3D reference model 20 (indicated with a second broken-line arrow) of the markers which is based on an initial spatial arrangement of the markers attached to the subject. The data processor 14 is configured to identify the markers and their positions. The data processor 14 is also configured to extract a marker arrangement based on the identified markers. The data processor 14 is further configured to search for a projection of the 3D reference model matching with a maximum subset of markers of the extracted marker arrangement. The data processor 14 is furthermore configured to identify markers of the extracted marker arrangement that are not part of the matching subset as temporarily ignored. The data processor 14 is configured to determine the searched projection of the 3D reference model that is matching with the maximum subset of the markers. The data processor 14 is also configured to identify a spatial orientation of the 3D reference model based on the projection direction. The output interface 16 is configured to provide the spatial orientation of the 3D reference model for a calculation of a spatial position of the subject.

For example, the provided image data is 2D image data or 3D image data.

The data output is indicated with a third broken-line arrow 22; the further data processing is indicated with a broken-line frame 24. The further data processing may be provided on another processing unit, as indicated, but may also be computed by the (same) data processor 14 as the other data processing operations.

The data input 12, the data processor 14 and the output interface 16 can be arranged within a common structure 26, like a housing, or in an integrated manner. However, it is also provided that the data input 12, the data processor 14 and the output interface 16 are arranged in a separated, but data-connected manner.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

The term "identifiable markers" relates to markers provided on the subject that can be tracked by tracking means. However, identifiable markers can also be provided as identified landmarks on the subject that are already existing and that can be tracked. Examples are skin marks, bone structures, implants or even tattoos.

The term "3D reference model of the markers" relates to a 3D model reflecting the spatial arrangement of the actual markers e.g. at an initial state. The 3D model is used as reference for the searching of the matching projection. The 3D model can be updated during the procedure, resulting in an updated 3D reference model of the markers.

As an option, the 3D model is provided based on image date representing the initial spatial situation of the markers as attached to the subject. For example, the image data is 2D image data. By providing two or more cameras, it is possible to retrieve spatial information even though the images are 2D.

In an example, a 3D positioning model is provided that provides a proposal for the arrangement of the markers on the subject. For example, a projection system is provided that provides visual projections for the correct locations on the subject such that a user is supported in finding proper location to achieve a marker arrangement without symmetry and with varying distances of the markers to each other.

The term "matching with a maximum subset of markers" relates to a projection in which the highest number of markers are matching. Non-matching of markers may be caused e.g. by shifted markers, removed markers or by artifacts.

The term "spatial position of the subject" relates to a position and orientation of the subject within the space, i.e. the spatial arrangement of the subject. The spatial position can be referring to an existing coordinate frame within the operation room, or to an existing coordinate frame of an imaging system, or to a coordinate frame of the subject.

In an example, the markers are provided as a set of stickers that are placed on the skin of the patient around the area of interest for surgery. The position of all stickers in the set can be combined to provide six degree-of-freedom (6DOF) tracking on the patient. For this purpose, a reference model of the marker set is stored at the start of the procedure. Patient tracking is carried out by comparing the current marker positions against the reference model.

When skin markers are used during a surgical procedure, the tracking system stores a reference model of the collection of markers at the start of the procedure. To determine the position and orientation of the subject, an observed set of markers is matched against that stored reference model. Due to noise in the observed positions, the matching is provided to allow for some variation. As an example for variation of marker positions, during surgery, markers can slightly move due to for example an incision made close to a marker. Each movement decreases the correlation of the set of markers to the reference model. Over time, the different can become so large that the subject position and orientation can no longer be established. If the difference with the reference model is too large when applying all markers, a reliable transformation cannot be computed. Due to identifying and excluding accidently moved markers, the matching and even transformation is possible.

In an option, not shown in detail, the data processor 14 is further configured to compute the spatial position of the subject based on the identified spatial orientation of the 3D reference model.

The position of the image detection arrangement during the acquisition of the image data is provided for the computing of the current spatial position. For example,

In an option, not shown in detail, the data input 12 is configured to provide image data comprising spatial information for the markers. The data processor 14 is configured to extract a 3D marker arrangement based on the identified markers and their positions and the spatial information for the markers; and to search for a spatial positioning of the 3D reference model matching with a maximum subset of markers of the extracted 3D marker arrangement; and to determine a spatial orientation of the 3D reference model for the searched spatial positioning that is matching with the maximum subset of the markers.

In a first option, the data input 12 is configured to provide image data comprising 2D images of the markers from at least two directions. The data processor 14 is configured to generate the 3D marker arrangement based on the 2D images of the markers from the at least two directions.

In a second option, the data input 12 is configured to provide image data comprising 3D image data of the markers. The data processor 14 is configured to generate the 3D marker arrangement based on the 3D image data of the markers.

In an option, not shown in detail, the data input 12 is configured to provide a 2D image. The data processor 14 is configured to extract a 2D marker arrangement based on the identified markers and their positions; and to search for a 2D projection of the 3D reference model matching with a maximum subset of markers of the extracted marker arrangement; and to identify the spatial orientation of the 3D reference model based on the searched 2D projection.

In an option, not shown in detail, the data input 12 is configured to provide image data, e.g. 2D or 3D image data, relating to at least one further point in time. The data processor 14 is further configured to repeat the steps of marker identification, marker extraction, search of a projection matching with a maximum subset of markers and determination of the searched projection for a calculation to track a movement of the subject, based on the image data relating to at least one further point in time.

As an example, image data, e.g. 2D or 3D image data, of a subject is provided from at least one point in time plus from a further, i.e. another point in time.

The term "further point in time" relates to a point in time that differs from the first point in time. In an example, the further point in time relates to a further stage during the same intervention or operation. In an example, several further points in time are identified for which points in time respective image data, e.g. 2D or 3D image data, is provided. In an example, image data is provided for several subsequent points in time, for example in a regular time pattern or with an irregular time pattern.

In an option, a more or less continuous stream of image data, e.g. 2D or 3D image data, is provided to be able to allow a more or less constant adapted tracking.

The term "repeat" relates to at least one further loop of the respective set of steps. In an option, several loops are provided. In a further option, a continuous arrangement of subsequent loops is provided. In another option, an update-loop is provided in regular or irregular intervals.

In an example, the identifying of moving markers allows to track the subject during breathing motion. As an example, breathing results in a non-rigid motion.

In another example, the identifying of moving markers allows to track the subject during heart motion. As an example, heart beating results in a non-rigid motion.

In an option, a plausibility check is provided as an extra step, e.g. applying a predetermined limit of deforming.

In an option, not shown in detail, the data processor 14 is further configured to compute a current spatial position of the subject based on the identified spatial orientation of the 3D reference model.

The term "current" relates to an actual spatial position, e.g. during an intervention, examination or other medical procedure. The tracking can be provided as live tracking.

For the comparing to the reference model, second tree branches are ignored to minimize computing effort.

In an option, not shown in detail, the provided image data of the subject relates to a first point in time. The data processor 14 is further configured to temporarily identify the temporarily ignored markers as displaced markers. The data input 12 is configured to provide image data from at least one further point in time. The data processor 14 is further configured to detect if the markers previously identified as temporarily ignored markers are having a stable position. The data processor 14 is also configured to identify stable markers of the temporarily ignored markers as shifted markers. The data processor 14 is furthermore configured to update the 3D reference model based on the shifted markers and to apply the updated 3D reference model for further projection searching steps.

The term "temporarily" relates to a situation or stage during which the placement of the marker is not verified. The time span can be several seconds or minutes, but can also be longer or shorter.

The term "displaced" relates to markers that cannot be identified as matching. The markers can be shifted, moved or even detached in relation to the subject.

The term "stable position" relates to markers identified and verified in their position at least over two subsequent images, e.g. subsequent 2D or 3D images.

The reference model of markers is updated if a marker moves slightly during the procedure, avoiding an eventual failure of the patient tracking functionality. For example, if an incision is made on one side of the subject, markers close to that incision may be temporarily ignored. Once their position is stable again, their new positions are updated in the reference model. Then, as a further option, incisions on the other side of the patient can be made, where the same set of events follows. At the end of the procedure, the entire reference model may be different than the one at the start, while still providing decent patient tracking.

Any observed markers that are not part of the latest match but close to markers in the reference model are marked as being in motion. Once a marker that is marked as in motion stops moving relative to the other markers (i.e. its movement is below a certain threshold) for a predetermined amount of time, the position of that marker in the reference model is updated. Tracking may resume based on the updated reference model corresponding to the full set of markers.

The movement of each marker relative to its original starting position is tracked over time. This is used to remove ambiguities between the motion of markers and patient movement. Any global transformations (rotations or translations) that are shared across this set of relative movements are removed. For example, if all markers have moved left by one millimeter, that is always considered patient movement and not movement of individual markers.

In an option, not shown in detail, for the provision of the 3D reference model, the data input 12 is configured to provide initial image data of the markers attached to the subject. The data processor 14 is further configured to determine the 3D model as an initial 3D model based on the initial image data, the 3D model representing an initial spatial arrangement of the markers.

The term "initial" relates to a state or point in time predefined, e.g. before an intervention takes place. However, initial can also refer to a point during an intervention. The tracking is provided after that initial point. In other words, the initial point in time is before the tracking or defines the start of the tracking or tracking period or tracking sequence.

The initial 3D model is then corrected based on the further image data.

In an option, it is provided to detect an incision based on the image data, e.g. 2D or 3D image data,.

Fig. 2 shows an example of an arrangement 30 of markers 32 as tracked by a tracking device. The arrangement 30 comprises at least four of the markers 32. In the example shown, eight of the markers 32 are provided, i.e. first marker 32a, second marker 32b, third marker 32c, fourth marker 32d, fifth marker 32e, sixth marker 32f, seventh marker 32g and eighth marker 32h. The markers 32 are attached to a subject, i.e. a patient, e.g. on the skin. The markers are therefore arranged in a three-dimensional pattern. However, when tracked by a tracking device, like a camera, the markers 32 are shown in a common image plane. (The numbering of the markers is provided for reference purposes only, and not for defining a certain order.)

The markers 32, as attached to the subject, are thus provided in a spatial arrangement with different distances between the individual markers. In Fig. 2, these distances are indicated with virtual connection lines 34, i.e. connection line 34-1, connection line 34-2, connection line 34-3, connection line 34-4, connection line 34-5, connection line 34-6, connection line 34-7, connection line 34-8, connection line 34-9, connection line 34-10, connection line 34-11, connection line 34-12 and connection line 34-13.

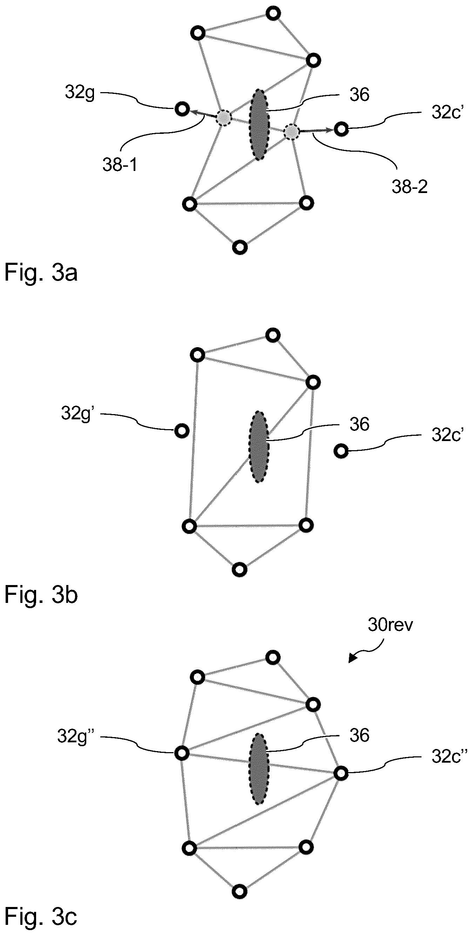

Fig. 3a, 3b and 3c show the arrangement 30 of the attached markers 32 in three different states illustrating an example of a scheme of subject tracking.

In Fig. 3a, while the markers 32 are attached e.g. to the subject's skin, an incision 36 is made by a surgeon or other medical staff member. This results in sort of deformation of the skin's surface. For example, parts of the skin along the incision 36 may be retracted due to skin surface tension. As a result, the seventh marker 32g, as tracked by the tracking device, moves, as indicated with a first displacement arrow 38-1 (to the left in the drawing) and the third marker 32c may also move, as indicated with a second displacement arrow 38-2 (to the right in the drawing).

The previous locations, indicated as the seventh marker 32g and the third marker 32c are shown in Fig. 3a for illustration purposes. The new locations are indicated as the displaced or moved seventh marker 32g' and the displaced or moved third marker 32c'.

When searching for a match of the current markers identified by the marker detection with a maximum number of markers of a projection of the reference model, the two moved markers, i.e. the moved seventh marker 32g' and the moved third marker 32c' are temporarily no longer considered for the match. In other words, they are temporarily de-selected.

In Fig. 3b, the de-selected markers, i.e. the moved seventh marker 32g' and the moved third marker 32c', are no longer shown as connected to the other markers by connecting lines. They are further tracked though in order to determine if their displacement action has come to a stop and if they are re-arranged in a new fixed location.

In Fig. 3c, the de-selected markers, i.e. the seventh marker, indicated as shifted seventh marker 32g", and the third marker, indicated as shifted third marker 32c", are again considered as temporarily fixed and are thus providing a new or revised marker arrangement 30ᵣₑᵥ. This new arrangement is then also used to update the reference model in order to be able to provide further tracking steps with searching for a match of maximum markers of further projections with the updated reference model.

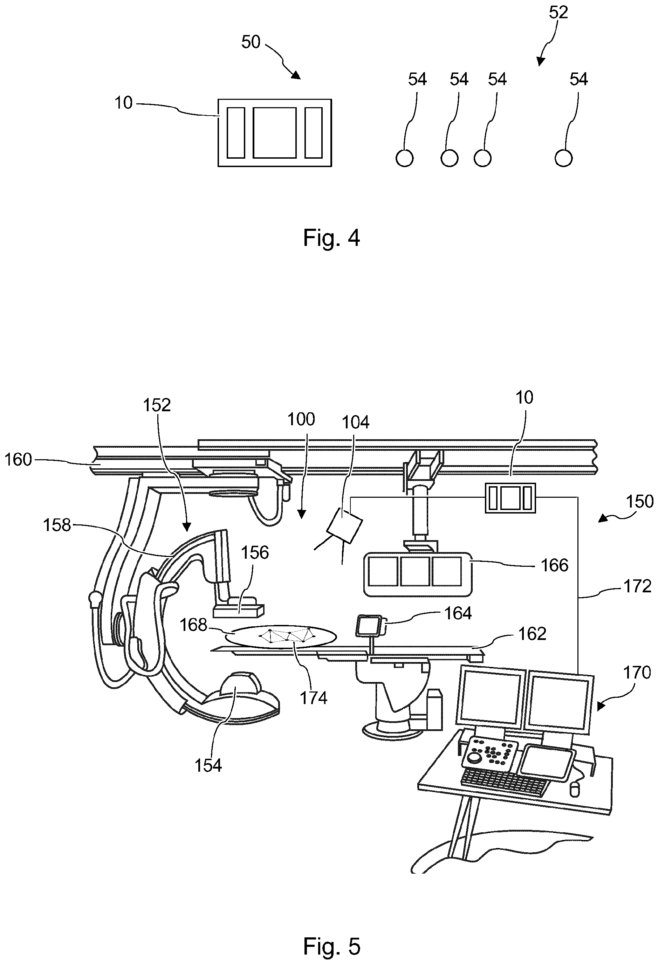

Fig. 4 shows an example of a subject tracking kit 50. The subject tracking kit 50 comprises an example of the subject tracking device 10 according to one of the examples above. The subject tracking kit 50 also comprises a set 52 of at least four markers 54. The markers 54 are configured to be attached to a subject's outer surface. The markers 54 are detectable by a marker detection device outputting image data to the subject tracking device, the image data comprising representations of the markers 54 attached to the subject.

As an example, the image data is 2D or 3D image data.

The outer surface comprises the subject's skin or hair. In an example, the outer surface comprises bone surfaces of the subject that are exposed.

In another example, the tracking method is also applicable for tracking non-human subjects, although the non-rigid part is typically less relevant in such cases.

In an option, not shown in detail, the markers 54 are markers detectable by a camera.

As an example, the markers 54 are optical markers detectable by an optical camera.

In another example, the markers are detectable by an infra-red camera.

In an option, at least a part of the markers are defined landmarks identifiable in the image data. As an example, the landmarks are distinctive skin portions or other visible landmarks of the subject.

In an option, not shown in detail, the markers 54 are detectable by at least one of the group comprising a depth camera using time-of-flight sensors, a LIDAR sensor and a structural light sensor.

In an option, not shown in detail, the markers 54 are electromagnetic markers detectable by an electromagnetic tracking system.

In an option, not shown in detail, the markers 54 are to be provided on the subject in a non-symmetric spatial arrangement. In another option, not shown in detail, provided in addition or as alternative, the markers 54 are to be provided on the subject with different distances between the markers 54.

In an example, the markers are to be provided in a non-symmetric spatial arrangement on the subject.

While the number of possible subsets for even a small number of markers can be intractably large, in an option, large branches from the search space can be cut off by evaluating distances between markers. If care is taken to avoid symmetries or similar distances during construction of the model, the remaining search space is sufficiently small.

In an option, for each subset that is evaluated, the pose is computed using the so-called *Umeyama* algorithm. The root mean square of the distances between the observed and reference markers is used estimate whether a match is correct. Searching for the larges matching subset handles the case of occluded markers, as these are not part of the set of observed markers. To guarantee a proper match is found using only a subset of markers, each possible subset must have a unique match to the reference model. This effectively means that the reference model should not have any symmetries. In an option, the user is instructed to change the pattern of stickers when symmetries are detected while creating the reference model.

False positives can lead to wrong results only if their position is such that together with the other markers, they form a pattern that matches part of the reference model. This is however extremely unlikely in practice and due to the temporal instability of most false positives is easily solved with temporal filtering.

Fig. 5 shows an example of a subject tracking system 100. The subject tracking system 100 comprises a marker detection arrangement 102 comprising at least one marker detection device 104. In a first option, the system 100 further comprises an example of the subject tracking device 10 according to one of the examples above. In a second option, the system 100 further comprises an example of the subject tracking kit 50 according to one of the examples above. The at least one marker detection device is configured to output image data, e.g. 2D or 3D image data, to the subject tracking device, the image data comprising representations of the markers attached to the subject.

In a first option, the subject tracking system 100 comprises the marker detection arrangement 102 and the subject tracking device 10. The marker detection arrangement 102 provides the image data to the subject tracking device 10.

In a second option, the subject tracking system 100 comprises the marker detection arrangement 102, the subject tracking device 10 and the set of markers. The marker detection arrangement 102 provides the image data to the subject tracking device 10.

Fig. 5 shows the subject tracking system 100 in the context of a medical intervention or examination context, e.g. an operation imaging arrangement 150. As an option, a medical imaging system is provided, like an X-ray system 152 with an X-ray source 154 and an X-ray detector 156 movably mounted to a C-arm 158. The C-arm can be movably suspended from a ceiling rail system 160. A subject support 162 is provided. A bedside control or operation interface 164 is shown, and a display arrangement 166 is also provided. A subject 168 is schematically indicated. Further, an operating or control console 170 is shown in the foreground, the console 170 comprising various kinds of user interfaces like keyboard, mouse, tablet, display and the like. The subject tracking device 10 is data-connected to the console 170, as indicated with data-connection line 172.

A marker arrangement 174 comprising at least four markers is attached to the subject 68.

In an example, the subject tracking system is integrated into a medical imaging system and the at least one marker detection device is provided by an imaging or detecting arrangement of the medical imaging system.

In an example, two or more marker detection devices are provided. For example, the marker detection devices are distributed across the operation room.

In an option, the marker detection device is provided as a camera based on visual light. In an option, the marker detection device is provided as a camera based on non-visual light, such as ultraviolet light or infrared light. In a further option, the marker detection device is provided as a time-of-flight or depth camera. In a still further option, the marker detection device is provided as a LIDAR sensor. In still another option, the marker detection device is provided as a structural light sensor.

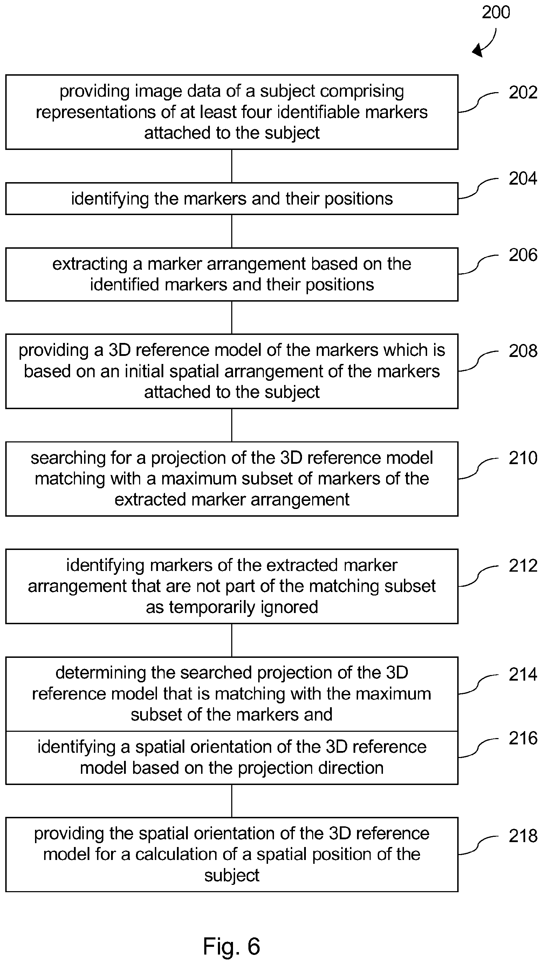

Fig. 6 shows basic steps of an example of a method 200 for subject tracking. The method 200 comprises the following steps:
- Image data of a subject is provided 202 comprising representations of at least four identifiable markers attached to the subject.
- The markers and their positions are identified 204.
- A marker arrangement is extracted 206 based on the identified markers and their positions.
- A 3D reference model of the markers is provided 208 which is based on an initial spatial arrangement of the markers attached to the subject.
- It is searched 210 for a projection of the 3D reference model matching with a maximum subset of markers of the extracted marker arrangement.
- Markers of the extracted marker arrangement that are not part of the matching subset are identified 212 as temporarily ignored.
- The searched projection of the 3D reference model that is matching with the maximum subset of the markers is determined 214 and a spatial orientation of the 3D reference model is identified 216 based on the projection direction.
- The spatial orientation of the 3D reference model is provided 218 for a calculation of a spatial position of the subject.

In an example, the step of identifying the markers comprise to identify their positions within the image plane.

In an example, at least seven markers are provided.

In an example of the method, it is further provided a step of computing the spatial position of the subject based on the determined searched projection.

In an example of the method, image data is provided relating to at least one further point in time; and the steps of marker identification, marker extraction, search of a projection matching with a maximum subset of markers and determination of the searched projection for a calculation are repeated to track a movement of the subject.

In an example of the method, a current spatial position of the subject is computed based on the identified spatial orientation of the 3D reference model.

In an example of the method, the provided image data of the subject relates to a first point in time. The temporarily ignored markers are temporarily identified as displaced markers. It is further provided the steps of:
- providing image data from at least one further point in time; and detecting if the markers previously identified as temporarily ignored markers are having a stable position;
- identifying stable markers of the temporarily ignored markers as shifted markers; and
- updating the 3D reference model based on the shifted markers and applying the updated 3D reference model for further projection searching steps.

As an example, the image data is 2D or 3D image data.

In an example of the method, the markers are configured to be attached to a subject's outer surface. In an option, the markers are detectable by a marker detection device outputting image data comprising representations of the markers attached to the subject.

In an example of the method, the markers are provided on the subject: i) in a non-symmetric spatial arrangement; and/or ii) with different distances between the markers.

In an example of the method, the markers provided in a non-symmetric spatial arrangement on the subject.

In an option, a guiding step is provided for attaching markers to the subject. As an example, the system detects markers and their spatial position and checks if these are according to the requirements, e.g. non-symmetric and with non-repeating distances to each other.

In another option, the user is guided by being provided with visual indicators that mark portions where the markers could be attached to the subject.

In an example of the method, for providing the 3D reference model, initial image data of the markers attached to the subject is provided; and the 3D model is determined as an initial 3D model based on the initial image data, the 3D model representing an initial spatial arrangement of the markers.

As an option, the marker tracking is applied in the context of a surgical navigation using augmented reality. Optical cameras and small stickers on the subject are used to track any patient movement. The system may be targeted for spine surgery. The many incisions that are made during the procedure, may gradually change the model formed by the set of subject markers. The present solution of robust marker tracking ensures to maintain patient tracking throughout the procedure.

As an example, a navigation system is provided that uses multiple optical markers. When moving one or more markers relative to the others, the system identifies this and continues to provide exact tracking. To avoid any unnecessary distraction for the operating staff members, the system does not trigger any warnings, or a recalibration procedure and the patient tracking remains active throughout the procedure.

The term "subject" may also be referred to as individual. The "subject" may further also be referred to as patient, although it is noted that this term does not indicate whether any illness or disease is actually present with the subject.

In an example, a computer program is provided comprising instructions which, when the program is executed by a computer, cause the computer to carry out the method of one of the examples above.

In an example, a computer program or program element for controlling an apparatus according to one of the examples above is provided, which program or program element, when being executed by a processing unit, is adapted to perform the method steps of one of the method examples above.

In an example, a computer readable medium is provided having stored the computer program of the example above.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for subject tracking, comprising:
- a data input (12);
- a data processor (14); and
- an output interface (16);
wherein the data input is configured: to provide image data (18) of a subject comprising representation of at least four identifiable markers attached to the subject; and to provide a 3D reference model (20) of the markers which is based on an initial spatial arrangement of the markers attached to the subject;
wherein the data processor is configured: to identify the markers and their positions; to extract a marker arrangement based on the identified markers and their positions;
to search for a spatial positioning of the 3D reference model matching with a maximum subset of markers of the extracted marker arrangement; to identify markers of the extracted marker arrangement that are not part of the matching subset as temporarily ignored; and
to determine the spatial orientation of the 3D reference model that is matching with the maximum subset of the markers; and
wherein the output interface is configured to provide the spatial orientation of the 3D reference model for a calculation of a spatial position of the subject.

2. Device according to claim 1, wherein the data processor is further configured to compute the spatial position of the subject based on the identified spatial orientation of the 3D reference model.

3. Device according to claim 1 or 2, wherein the data input is configured to provide image data comprising spatial information for the markers;
wherein the data processor is configured to extract a 3D marker arrangement based on the identified markers and their positions and the spatial information for the markers; and to search for a spatial positioning of the 3D reference model matching with a maximum subset of markers of the extracted 3D marker arrangement; and to determine a spatial orientation of the 3D reference model for the searched spatial positioning that is matching with the maximum subset of the markers; and
wherein:
i) the data input is configured to provide image data comprising 2D images of the markers from at least two directions; and wherein the data processor is configured to generate the 3D marker arrangement based on the 2D images of the markers from the at least two directions; or
ii) the data input is configured to provide image data comprising 3D image data of the markers; and wherein the data processor is configured to generate the 3D marker arrangement based on the 3D image data of the markers.

4. Device according to claim 1 or 2, wherein the data input is configured to provide a 2D image; and
wherein the data processor is configured to extract a 2D marker arrangement based on the identified markers and their positions; and to search for a 2D projection of the 3D reference model matching with a maximum subset of markers of the extracted marker arrangement; and to identify the spatial orientation of the 3D reference model based on the searched 2D projection.

5. Device according to one of the preceding claims, wherein the data input is configured to provide image data relating to at least one further point in time; and
wherein the data processor is further configured to repeat the steps of marker identification, marker extraction, search of a projection matching with a maximum subset of markers and determination of the searched projection for a calculation to track a movement of the subject, based on the image data relating to at least one further point in time.

6. Device according to one of the preceding claims, wherein the data processor is further configured to compute a current spatial position of the subject based on the identified spatial orientation of the 3D reference model.

7. Device according to one of the preceding claims, wherein the provided image data of the subject relates to a first point in time;
wherein the data processor is further configured to temporarily identify the temporarily ignored markers as displaced markers;
wherein the data input is configured to provide image data from at least one further point in time; and
wherein the data processor is further configured: to detect if the markers previously identified as temporarily ignored markers are having a stable position; to identify stable markers of the temporarily ignored markers as shifted markers; and to update the 3D reference model based on the shifted markers and to apply the updated 3D reference model for further projection searching steps.

8. Device according to one of the preceding claims, wherein, for the provision of the 3D reference model, the data input is configured to provide initial image data of the markers attached to the subject; and
wherein the data processor is further configured to determine the 3D model as an initial 3D model based on the initial image data, the 3D model representing an initial spatial arrangement of the markers.

9. A subject tracking kit (50), comprising:
- a subject tracking device (10) according to one of the claims 1 to 8; and
- a set of at least four markers (54);
wherein the markers are configured to be attached to a subject's outer surface; and
wherein the markers are detectable by a marker detection device outputting image data to the subject tracking device, the image data comprising representations of the markers attached to the subject.

10. Kit according to claim 9, wherein the markers are markers detectable by:
i) a camera; or
ii) at least one of the group comprising:
- a depth camera using time-of-flight sensors;
- a LIDAR sensor; and
- a structural light sensor.

11. Kit according to claim 9 or 10, wherein the markers are electromagnetic markers detectable by an electromagnetic tracking system.

12. Kit according to one of claims 9 to 11, wherein the markers are to be provided on the subject:
i) in a non-symmetric spatial arrangement; and/or
ii) with different distances between the markers.

13. A subject tracking system (100), comprising:
- a marker detection arrangement (102) comprising at least one marker detection device (104); and
i) a subject tracking device (10) according to one of the claims 1 to 8; or
ii) a subject tracking kit (50) according to one of the claims 9 to 12;
wherein the at least one marker detection device is configured to output image data to the subject tracking device, the image data comprising representations of the markers attached to the subject.

14. A method (200) for subject tracking, the method comprising the following steps:
- providing (202) image data of a subject comprising representations of at least four identifiable markers attached to the subject;
- identifying (204) the markers and their positions;
- extracting (206) a marker arrangement based on the identified markers and their positions;
- providing (208) a 3D reference model of the markers which is based on an initial spatial arrangement of the markers attached to the subject;
- searching (210) for a projection of the 3D reference model matching with a maximum subset of markers of the extracted marker arrangement;
- identifying (212) markers of the extracted marker arrangement that are not part of the matching subset as temporarily ignored;
- determining (214) the searched projection of the 3D reference model that is matching with the maximum subset of the markers and identifying (216) a spatial orientation of the 3D reference model based on the projection direction; and
- providing (218) the spatial orientation of the 3D reference model for a calculation of a spatial position of the subject.

15. A computer program comprising instructions which, when the program is executed by a computer, cause the computer to carry out the method of claim 14.
